# EUROPEAN PATENT APPLICATION

(11) **EP 3 138 606 A1**
(43) Date of publication of application: **08.03.2017**
(21) Application number: 15785671.7
(22) Date of filing: 28.04.2015
(51) Int. Cl.: A61N 5/06, A61B 18/00

(54) **KIT FOR LIPOLYSIS BY MEANS OF LIGHT RADIATION**

(30) Priority: 30.04.2014 KR 20140052315
(71) Applicant: Amorepacific Corporation, Seoul 140-777 (KR)
(72) Inventor: CHOI, Min Sik, Yongin-si Gyeonggi-do 446-729 (KR); KIM, Hyoung June, Yongin-si Gyeonggi-do 446-729 (KR); JUNG, Ji Yong, Yongin-si Gyeonggi-do 446-729 (KR); LEE, Tae Ryong, Yongin-si Gyeonggi-do 446-729 (KR); SHIN, Dong Wook, Yongin-si Gyeonggi-do 446-729 (KR)
(74) Representative: Bugnion Genève
(86) International application number: PCT/KR2015/004236
(87) International publication number: WO 2015/167213

(57) **Abstract**

This specification provides a light source radiation apparatus for radiating light, a kit for lipolysis having a manual describing a method for dissolving fat by radiating light, and a slimming method for radiating light to skin. According to the specification, the present invention can dissolve neutral fat in a fat cell by radiating light of a wavelength in a range of light harmless to the body, thus can safely and effectively dissolve fat, and can be used for the slimming method by having the advantage of being more economical and convenient than a conventional lipolysis method.

## Description

### [Technical Field]

The present disclosure relates to a kit for lipolysis and a slimming method.

### [Background Art]

Obesity results from accumulation of excess energy in adipocytes as triglycerides. Prevention and improvement of obesity are strongly required because it can cause various diseases comprising arteriosclerosis and is aesthetically unfavorable.

Recently, people with obesity are increasing every year worldwide, particularly in developed countries, due to overeating, lack of exercise, stress, etc. They often drop out of dieting or exercise because it requires strong will and long time.

Therefore, efforts are made to develop a method for lipolysis or an agent facilitating lipolysis in order to prevent and improve obesity. For example, although the ingredients obtained from natural extracts are used to facilitate lipolysis, the effect is not necessarily sufficient and some of them have undesired side effects. Meanwhile, there have been few researches on the facilitation of lipolysis in the level of adipocytes.

### [Disclosure]

### [Technical Problem]

The present disclosure is directed to providing a kit for lipolysis which degrades fats in adipocytes by irradiating light to adipocytes in skin or subcutaneous fat layer.

### [Technical Solution]

In an aspect, the present disclosure provides a kit for lipolysis comprising: a light irradiation device which irradiates light; and an instruction which describes a method of degrading fat by irradiating light.

In an exemplary embodiment of the present disclosure, the light may have a wavelength which is longer than 600 nm and equal to or shorter than 1500 nm.

In an exemplary embodiment of the present disclosure, the light may be visible light or infrared light.

In an exemplary embodiment of the present disclosure, the light may have a pattern which enhances skin penetration.

In an exemplary embodiment of the present disclosure, the instruction may comprise light irradiation intensity, light irradiation time, the distance between the irradiation device and skin, irradiation method and irradiated part.

In an exemplary embodiment of the present disclosure, the light irradiation intensity may be 1-600 J/cm².

In an exemplary embodiment of the present disclosure, the light irradiation time may be from 0.5 minute to 6 hours. In an exemplary embodiment of the present disclosure, the distance between the irradiation device and skin may be 0-200 cm.

In an exemplary embodiment of the present disclosure, the irradiation may be performed by direct irradiation.

In an exemplary embodiment of the present disclosure, the irradiation may be performed using an auxiliary means that can be contacted to skin.

In an exemplary embodiment of the present disclosure, the auxiliary means may be one or more selected from a group consisting of a filter, a sheet, a film, a cosmetic, a gel, a cream, etc.

In an exemplary embodiment of the present disclosure, the irradiation may be performed using one or more selected from a group consisting of a needle tube, a cannula and a microneedle.

In an exemplary embodiment of the present disclosure, the irradiated part may be any skin tissue where fat can be accumulated, excluding eyes, mouth, sexual organ and anus.

The present disclosure also provides a slimming method comprising irradiating light to skin.

In an exemplary embodiment of the present disclosure, the light may have an intensity of 1-600 J/cm².

In an exemplary embodiment of the present disclosure, the irradiation may be performed for from 0.5 minute to 6 hours.

In an exemplary embodiment of the present disclosure, the irradiation may be performed at a distance of 0-200 cm from skin.

In an exemplary embodiment of the present disclosure, the irradiation may be performed by direct irradiation.

In an exemplary embodiment of the present disclosure, the irradiation may be performed using an auxiliary means that can be contacted to skin.

In an exemplary embodiment of the present disclosure, the irradiation may be performed onto the subcutaneous fat layer of skin.

In an exemplary embodiment of the present disclosure, the subcutaneous fat layer of skin may comprise undifferentiated, differentiating or differentiated adipocytes.

In an exemplary embodiment of the present disclosure, the irradiation may degrade the triglycerides of adipocytes to glycerol.

### [Advantageous Effects]

According to the present disclosure, fat can be degraded safely and effectively because triglycerides in adipocytes can be degraded by irradiating light in a wavelength range unharmful to the body. The present disclosure can be used for slimming because it is more economical and convenient than the existing lipolysis method.

### [Brief Description of Drawings]

FIG. 1 shows a result of irradiating lights of specific wavelengths to adipocytes in different differentiation stages and measuring total triglyceride (TG) and serum glycerol levels according to an exemplary embodiment of the present disclosure.
FIG. 2 shows a result of irradiating lights of specific wavelengths to differentiated adipocytes and measuring total triglyceride and serum glycerol levels according to an exemplary embodiment of the present disclosure.
FIG. 3 shows a result of irradiating lights of specific wavelengths to differentiated adipocytes and measuring triglyceride (TG) contents in the adipocytes according to an exemplary embodiment of the present disclosure.
FIG. 4 exemplarily shows wavelengths and λₘₐₓ's of lights.

### [Best Mode]

Korean Patent Application No. 10-2014-0052315, filed on April 30, 2014, is incorporated in the present disclosure in its entirety by reference. Also, this application claims the priority of Korean Patent Application No. 10-2014-0052315, the contents of which in its entirety is incorporated herein by reference.

Hereinafter, specific embodiments of the present disclosure are described in detail such that those of ordinary skill in the art to which the present disclosure belongs can easily carry out the present disclosure.

The present disclosure provides a kit for lipolysis comprising: a light irradiation device which irradiates light; and an instruction which describes a method of degrading fat by irradiating light.

In the present disclosure, the light may be visible light or infrared light.

In an aspect of the present disclosure, the light irradiation device may comprise: a power supply; an input; a light source; a sensor; a controller; and a display, although not being limited thereto.

In an aspect of the present disclosure, the input may be one for inputting light intensity, irradiation time, light wavelength and light pattern required for irradiating light. The light source may generate and irradiate light. The sensor may be of various types and may be one for measuring the distance between the irradiation device and skin. The controller may control the light irradiation by the light source as inputted in the input. The display may display the inputted data and the distance to skin.

In an aspect of the present disclosure, the light source may comprise a sunlight permeable material. In an aspect of the present disclosure, the sunlight permeable material may be any one that can allow light to pass therethrough. For example, a quartz plate, a band-pass filter which passes light within a certain range, etc. may be used, although not being limited thereto.

In an aspect of the present disclosure, the light source may be a light-emitting diode (LED), an organic light-emitting diode (OLED), a laser diode (LD), a laser, an electrodeless lamp, a carbon nanotube (CNT), a cold-cathode fluorescent lamp (CCFL), a flat fluorescent lamp (FFL), an external electrode fluorescent lamp (EEFL), an incandescent lamp, a halogen lamp, a fluorescent lamp, a tungsten lamp, a low-pressure/high-pressure sodium lamp, a low-pressure/high-pressure mercury lamp, a xenon lamp, a metal halide lamp, an HID lamp, a remote light source (e.g., optical fiber or prism light), a xenon arc lamp, etc., although not being limited thereto.

In the present disclosure, the light irradiation device means any device that can emit light. A device which is not originally intended for irradiation of light but can be easily modified by those of ordinary skill in the art to which the present disclosure belongs to irradiate light is also comprised. The light irradiation device may also be a light therapy device used in hospitals or oriental medicine clinics or a personal or hand-held light irradiation device.

In an exemplary embodiment of the present disclosure, the instruction may comprise light irradiation intensity, light irradiation time, the distance between the irradiation device and skin, irradiation method and irradiated part. In an aspect of the present disclosure, the instruction may comprise information for control of one or more of the light irradiation intensity, irradiation time and distance from skin depending on the user's condition.

In an exemplary embodiment of the present disclosure, the light irradiation intensity may be 1-600 J/cm². In an exemplary embodiment of the present disclosure, the light irradiation time may be from 0.5 minute to 6 hours. When the irradiation intensity is lower than 1 J/cm² or when the irradiation time is shorter than 0.5 minute, the effect obtained from the irradiation may be insignificant. And, when the irradiation intensity is higher than 600 J/cm² or when the irradiation time is longer than 6 hours, the irradiation may cause skin damage. In the present disclosure, the light irradiation intensity may be 1 J/cm² or higher, 10 J/cm² or higher, 50 J/cm² or higher, 100 J/cm² or higher, 150 J/cm² or higher, 200 J/cm² or higher or 250 J/cm² or higher, although not being limited thereto. In the present disclosure, the light irradiation intensity may be 600 J/cm² or lower, 550 J/cm² or lower, 500 J/cm² or lower, 450 J/cm² or lower, 400 J/cm² or lower, 350 J/cm² or lower or 300 J/cm² or lower, although not being limited thereto. In the present disclosure, the light irradiation time may be 10 minutes or longer, 30 minutes or longer, 1 hour or longer, 1 hour and 30 minutes or longer, 2 hours or longer or 2 hours and 30 minutes or longer, although not being limited thereto. In the present disclosure, the light irradiation time may be 5 hours and 30 minutes or shorter, 5 hours or shorter, 4 hours and 30 minutes or shorter, 4 hours or shorter, 3 hours and 30 minutes or shorter or 3 hours or shorter, although not being limited thereto.

The distance between the irradiation device and skin means the distance between the light source of the irradiation device and skin measured by the sensor of the irradiation device. In an exemplary embodiment of the present disclosure, the distance between the irradiation device and skin may be 0-200 cm. When the distance from skin is shorter than 0 cm, it means that the device is not contacted to skin but penetrates the skin. And, when the distance exceeds 200 cm, the effect obtained from the irradiation may be insignificant. In the present disclosure, the distance between the irradiation device and skin may be 5 cm or longer, 10 cm or longer, 15 cm or longer, 20 cm or longer, 25 cm or longer, 30 cm or longer, 35 cm or longer, 40 cm or longer or 45 cm or longer, although not being limited thereto. In the present disclosure, the distance between the irradiation device and skin may be 170 cm or shorter, 140 cm or shorter, 110 cm or shorter, 80 cm or shorter, 75 cm or shorter, 70 cm or shorter, 65 cm or shorter, 60 cm or shorter or 55 cm or shorter, although not being limited thereto.

The irradiation may be performed using one or more auxiliary means selected from a group consisting of a filter, a sheet, a film, a cosmetic, a gel and a cream. Also, the irradiation may be performed using one or more selected from a group consisting of a needle tube, a cannula and a microneedle.

The irradiated part may be any skin tissue where fat can be accumulated, excluding eyes, mouth, sexual organ and anus.

The present disclosure also provides a slimming method comprising irradiating light to skin or its subcutaneous fat layer.

In the present disclosure, the "slimming" may mean reducing the circumference or thickness of the body or a part of it with respect to the height or length. Also, the "slimming" may mean making the body or a part of it slender or losing body weight.

In an exemplary embodiment of the present disclosure, the adipocytes refer to common fat cells and are not limited as long as they are cells that store fat. They comprise all of undifferentiated, differentiating and differentiated cells.

In an exemplary embodiment of the present disclosure, the skin comprises the epidermis, the dermis and the subcutaneous fat layer. The adipocytes present in each layer may be the target of light irradiation in the present disclosure.

In an exemplary embodiment of the present disclosure, the light may be light of any wavelength. And, the light may be free from heat. Lipolysis can be facilitated with light alone.

In the present disclosure, the irradiated light may have a wavelength which is longer than 600 nm and equal to or shorter than 1500 nm. In the present disclosure, the irradiated light may have a wavelength which is longer than 600 nm, 605 nm or longer, 610 nm or longer, 615 nm or longer, 620 nm or longer, 625 nm or longer, 630 nm or longer, 640 nm or longer, 641 nm or longer, 643 nm or longer, 645 nm or longer, 647 nm or longer, 649 nm or longer, 650 nm or longer, 660 nm or longer, 670 nm or longer, 680 nm or longer, 690 nm or longer, 700 nm or longer, 710 nm or longer, 730 nm or longer, 750 nm or longer, 770 nm or longer, 790 nm or longer, 800 nm or longer, 850 nm or longer, 900 nm or longer, 950 nm or longer, 1000 nm or longer, 1050 nm or longer, 1100 nm or longer, 1150 nm or longer, 1200 nm or longer, 1250 nm or longer, 1300 nm or longer, 1350 nm or longer, 1400 nm or longer, 1450 nm or longer or 1500 nm or longer. In the present disclosure, the irradiated light may have a wavelength which is 2000 nm or shorter, 1600 nm or shorter, 1500 nm or shorter, 1450 nm or shorter, 1400 nm or shorter, 1350 nm or shorter, 1300 nm or shorter, 1250 nm or shorter, 1200 nm or shorter, 1150 nm or shorter, 1100 nm or shorter, 1050 nm or shorter, 1000 nm or shorter, 950 nm or shorter, 900 nm or shorter, 850 nm or shorter, 800 nm or shorter, 790 nm or shorter, 770 nm or shorter, 750 nm or shorter, 730 nm or shorter, 710 nm or shorter, 700 nm or shorter, 690 nm or shorter, 680 nm or shorter, 670 nm or shorter, 660 nm or shorter, 650 nm or shorter, 648 nm or shorter, 646 nm or shorter, 644 nm or shorter, 642 nm or shorter, 640 nm or shorter, 635 nm or shorter, 630 nm or shorter, 625 nm or shorter, 620 nm or shorter, 615 nm or shorter, 610 nm or shorter, 605 nm or shorter or 601 nm or shorter. Most specifically, in the present disclosure, the light may have a wavelength which is longer than 600 nm and equal to or shorter than 700 nm.

Also, in an aspect of the present disclosure, the light may be one having the above-described wavelength as a maximum energy wavelength (λ_{may}). Specifically, in an aspect of the present disclosure, the light may have a wavelength in the range of λₘₐₓ ± 100 nm. More specifically, in an aspect of the present disclosure, the light may have a wavelength in the range of λₘₐₓ ± 90 nm or less, λₘₐₓ ± 80 nm or less, λₘₐₓ ± 70 nm or less, λₘₐₓ ± 60 nm or less, λₘₐₓ ± 50 nm or less, λₘₐₓ ± 40 nm or less, λₘₐₓ ± 30 nm or less, λₘₐₓ ± 20 nm or less, λₘₐₓ ± 10 nm or less or λₘₐₓ ± 5 nm or less. For example, in an aspect of the present disclosure, the light may have a wavelength which is longer than 600 nm and equal to or shorter than 700 nm when λₘₐₓ is 660 nm; a wavelength of 700-800 nm when λₘₐₓ is 740 nm; a wavelength of 800-900 nm when λₘₐₓ is 850 nm; a wavelength of 900-1,000 nm when λₘₐₓ is 940 nm; a wavelength of 1,000-1,100 nm when λₘₐₓ is 1,050 nm; a wavelength of 1,100-1,200 nm when λₘₐₓ is 1,150 nm; a wavelength of 1,200-1,300 nm when λₘₐₓ is 1,250 nm; and a wavelength of 1,300-1,400 nm when λₘₐₓ is 1,350 nm.

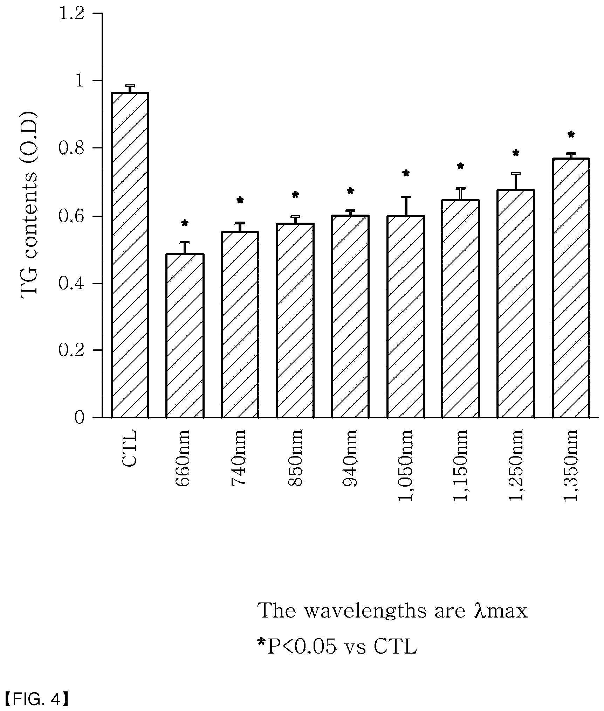

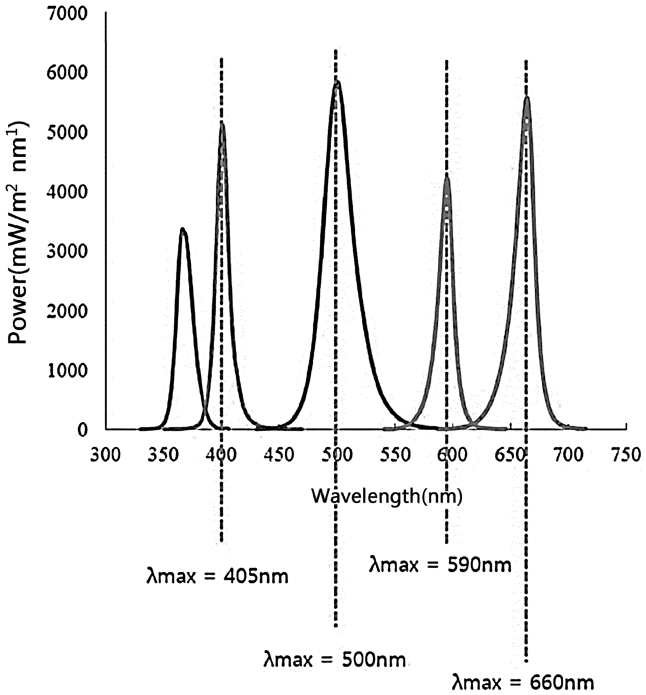

In the present disclosure, the maximum energy wavelength, or λₘₐₓ, means the wavelength where the energy of light is the highest in the given wavelength range. Specifically, for a bell-shaped normal distribution with wavelength as the x-axis and light energy as the y-axis, λₘₐₓ means the wavelength where the energy is the highest (peak). For example, in FIG. 4, the wavelengths marked by dotted lines are λₘₐₓ's for the given wavelength ranges.

In an aspect of the present disclosure, the irradiation may suppress fat formation or degrade fats in undifferentiated or differentiating adipocytes and may also degrade fats in differentiated adipocytes. The light irradiation to undifferentiated, differentiating or differentiated adipocytes provides the effect of suppressing fat formation or degrading fats. And, the light irradiation to skin or its subcutaneous fat layer provides lipolytic effect for various adipocytes present in the skin which are in different stages of differentiation.

When the adipocytes are undifferentiated cells, the irradiation may be performed on skin or its subcutaneous fat layer prior to or during differentiation of the adipocytes. When the irradiation is performed during differentiation, it may be performed in any stage. The irradiation may be performed only once or 2 or more times.

Specifically, a step of irradiating light undifferentiated adipocytes cells in a phosphate-buffered saline (PBS) medium; a step of culturing the adipocytes in a cocktail medium for differentiation for 1-3 days and then irradiating light again in a phosphate-buffered saline (PBS) medium; a step of culturing the adipocytes in an insulin medium for 2-4 days and then irradiating light again in a phosphate-buffered saline (PBS) medium; and a step of culturing the adipocytes in a fetal bovine serum (FBS) medium for 1-3 days may be comprised, although not being limited thereto.

Also, a step of culturing undifferentiated adipocytes cells for 1-3 days and then irradiating light in a phosphate-buffered saline (PBS) medium; and a step of culturing the adipocytes in a cocktail medium for differentiation for 1-3 days, culturing them in an insulin medium for 2-4 days and then culturing them in a fetal bovine serum (FBS) medium for 1-3 days may be comprised, although not being limited thereto.

Also, a step of culturing undifferentiated adipocytes cells for 1-3 days, culturing them in a cocktail medium for differentiation for 1-3 days and then irradiating light in a phosphate-buffered saline (PBS) medium; and a step of culturing the adipocytes in an insulin medium for 2-4 days and then culturing them in a fetal bovine serum (FBS) medium for 1-3 days may be comprised, although not being limited thereto.

Also, a step of culturing undifferentiated adipocytes cells for 1-3 days, culturing them in a cocktail medium for differentiation for 1-3 days, culturing them in an insulin medium for 2-4 days and then irradiating light in a phosphate-buffered saline (PBS) medium; and a step of culturing the adipocytes in a fetal bovine serum (FBS) medium for 1-3 days may be comprised, although not being limited thereto.

Through this, not only fats in differentiated adipocytes can be degraded but also fat formation during differentiation of undifferentiated cells can be suppressed, as demonstrated in the test examples described below.

Also, the irradiation may be performed after the differentiation of adipocytes has been completed.

Specifically, a step of culturing undifferentiated adipocytes cells for 1-3 days, culturing them in a cocktail medium for differentiation for 1-3 days, culturing them in an insulin medium for 2-4 days and then culturing them in a fetal bovine serum (FBS) medium for 1-3 days; and a step of irradiating light to the adipocytes in a phosphate-buffered saline (PBS) medium may be comprised, although not being limited thereto.

That is to say, undifferentiated adipocytes cells are differentiated as they are cultured in the cocktail medium, the insulin medium and the FBS medium and then light is irradiated to the differentiated cells.

When light is irradiated to the differentiated adipocytes, the degradation of fats produced in the adipocytes can be facilitated, as demonstrated in the test examples described below.

In the present disclosure, the cocktail medium for differentiation is not particularly limited as long as it induces transition of cellular proliferation to differentiation. For example, it may be a medium comprising one or more selected from a group consisting of fetal bovine serum (FBS), insulin, 3-isobutyl-1-methylxanthine (IBMX) and dexamethasone (DEX). The concentration of each ingredient can be varied depending on the differentiation condition of adipocytes. Specifically, 4.5 g/L glucose-comprising DMEM supplemented with 10% fetal bovine serum (FBS), 10 mg/mL insulin, 0.5 mM 3-isobutyl-1-methylxanthine (IBMX) and 1 mM dexamethasone (DEX) may be used.

In the present disclosure, the insulin medium serves to form lipid droplets (envelopes) in the adipocytes and the FBS medium serves to supply energy by filling fats in the lipid droplets.

In the present disclosure, the irradiation may be performed when the adipocytes are present in a phosphate-buffered saline (PBS) medium, although not being specially limited thereto. It is because it is important to irradiate light to the adipocytes without special limitation in order to facilitate lipolysis. When a commonly used medium is used, light of a specific wavelength may not be fully transmitted to the adipocytes due to interference, scattering, etc. of light by the various ingredients comprised in the medium. In contrast, the phosphate-buffered saline (PBS) medium is superior in transmitting light of a specific wavelength because the interference, scattering, etc. are prevented.

In the present disclosure, the light irradiation may be performed in a non-invasive or invasive manner. The non-invasive irradiation is a method of irradiating light directly onto skin, such that the irradiated light reaches the adipocytes present at the shallow depth of the skin such as the epidermis, dermis, etc. depending on the wavelength of the light. The invasive irradiation is not particularly limited as long as it is a known method. For example, a needle tube, a cannula, a microneedle, etc. may be inserted into skin and then light may be irradiated such that the light reaches the adipocytes present at a depth of, e.g., 5 mm or longer from the epidermis.

Also, when irradiating light according to the present disclosure, an auxiliary means which can increase the transmission of the light to the adipocytes may be applied to skin. The auxiliary means may be one that can be contacted to skin and the light irradiation according to the present disclosure may be performed using the auxiliary means. Also, when irradiating light according to the present disclosure, a light having a pattern which enhances skin penetration may be used to increase the transmission of the light to the adipocytes.

In the present disclosure, the irradiation of light with a specific wavelength degrades the triglycerides of adipocytes to glycerol. A lipolytic effect is achieved as the triglycerides are degraded.

According to the present disclosure, fat can be degraded safely and effectively because the triglycerides in adipocytes can be degraded by irradiating light in a wavelength range unharmful to the body. The present disclosure can be used for slimming because it is more economical and convenient than the existing lipolysis method.

Hereinafter, the present disclosure will be described in detail through examples. However, the following examples are for illustrative purposes only and the scope of the present disclosure is not limited by the examples.

### [Example 1]

### Light irradiation throughout whole stages

### 1-1. Culturing of undifferentiated adipocytes

Undifferentiated mouse 3T3-L1 fibroblasts were acquired from ATCC (CL-173). The undifferentiated cells (preadipocytes) were cultured for 2 days in a humid incubator comprising 10% CO₂ using 4.5 g/L glucose-comprising DMEM (Dulbecco's modified Eagle's medium) (PAA, Austria) supplemented with 10% calf serum (Gibco BRL, NY, USA).

### 1-2. Differentiation into adipocytes

For differentiation of the undifferentiated adipocytes obtained in 1-1, the medium was replaced with 4.5 g/L glucose-comprising DMEM supplemented with 10% fetal bovine serum (FBS; PAA, Austria), 10 mg/mL insulin (Sigma-Aldrich, St. Louis, USA), 0.5 mM 3-isobutyl-1-methylxanthine (IBMX; Sigma-Aldrich, St. Louis, USA) and 1 mM dexamethasone (DEX; Sigma-Aldrich, St. Louis, USA), as a cocktail medium for differentiation. After culturing for 2 days, the medium was replaced with 4.5 g/L glucose-comprising DMEM supplemented with 10% FBS and 10 mg/mL insulin and then the cells were cultured for 3 days. Then, the cells were cultured for 2 days in insulin-free, 4.5 g/L glucose-comprising DMEM supplemented with 10% FBS.

### 1-3. Light irradiation

1) The cultured undifferentiated cells (preadipocytes) in obtained in 1-1 were washed 2 times with phosphate-buffered saline (PBS; CaCl₂- and MgCl₂-free, Welgene, Korea) before exchanging the medium with the cocktail medium. Then, the cells were immersed in fresh PBS and irradiated with light with λₘₐₓ of 660 nm, 740 nm, 850 nm, 940 nm, 1,050 nm, 1,150 nm, 1,250 nm or 1,350 nm for 1 hour in a humid incubator comprising 10% CO₂. During the light irradiation, the irradiation distance was 5 cm and the irradiation intensity was 10 J/cm². A control (CTL) was treated under the same condition except for the light irradiation.
2) After the light irradiation, the medium was replaced with the cocktail medium used in 1-2 and the cells were cultured for 2 days.
3) The cells that had been cultured for 48 hours in the cocktail medium were washed 2 times with PBS and irradiated with the light of the same wavelength as in 1) for 1 hour after being immersed in PBS. After replacing the medium with an insulin medium, the cells were cultured for 3 days.
4) The cells that had been cultured for 3 days in the insulin medium were washed 2 times with PBS and irradiated with the light of the same wavelength as in 1) for 1 hour after being immersed in PBS. After replacing the medium with an FBS medium, the cells were cultured for 3 days. The cells were washed 2 times with PBS and irradiated with the light of the same wavelength as in 1) for 1 hour after being immersed in PBS

### 1-4. Recovery of medium for measuring triglycerides and glycerol

After the light irradiation in 1-3, the cultured cells were washed 2 times with PBS. In order to measure total triglycerides and glycerol eluted into a medium, the cells were cultured for 6 hours in 1,000 mg/L glucose-comprising, nutrient-restricted DMEM (PAA, Austria) supplemented with 2% bovine serum albumin (BSA; Sigma-Aldrich, St. Louis, USA). Then, the medium was recovered from the control group (CTL) and the test groups which had been treated with lights with λₘₐₓ of 660 nm, 740 nm, 850 nm, 940 nm, 1,050 nm, 1,150 nm, 1,250 nm and 1,350 nm.

### [Example 2]

### Light irradiation after differentiation

### 2-1. Culturing of undifferentiated adipocytes

Undifferentiated cells were obtained in the same manner as in Example 1-1.

### 2-2. Differentiation into adipocytes

The undifferentiated cells were differentiated into adipocytes in the same manner as in Example 1-2.

### 2-3. Light irradiation

The differentiated adipocytes obtained in 2-2 were immersed in PBS and irradiated with light with λₘₐₓ of 660 nm, 740 nm, 850 nm, 940 nm, 1,050 nm, 1,150 nm, 1,250 nm or 1,350 nm for 6 hours in the same nutrient-restricted medium as in 1-4. During the light irradiation, the irradiation distance was 5 cm and the irradiation intensity was 10 J/cm².

### 2-4. Recovery of medium for measuring triglycerides and glycerol

The medium was recovered from the control group (CTL) and the test groups which had been treated with lights with λₘₐₓ of 660 nm, 740 nm, 850 nm, 940 nm, 1,050 nm, 1,150 nm, 1,250 nm and 1,350 nm in the same manner as in Example 1-4.

### [Test Example 1] Measurement of total triglycerides and glycerol eluted from cells into medium

The amount of triglycerides and glycerol eluted into the nutrient-restricted medium which was recovered after the light irradiation in Examples 1 and 2 was measured using Triglyceride Reagent (Sigma-Aldrich, USA) and Free Glycerol Reagent (Sigma-Aldrich, USA). The amount of glycerol was determined by measuring the medium with Free Glycerol Reagent and measuring absorbance at a wavelength of 540 nm after incubation for 15 minutes. The amount of eluted triglycerides was measured indirectly using a lipase. First, the eluted triglycerides were degraded into glycerol and fatty acids using a lipase and the amount of glycerol was measured. By subtracting the amount of glycerol that had been eluted prior to the lipase treatment from this measured value, the amount of the eluted triglycerides was calculated.

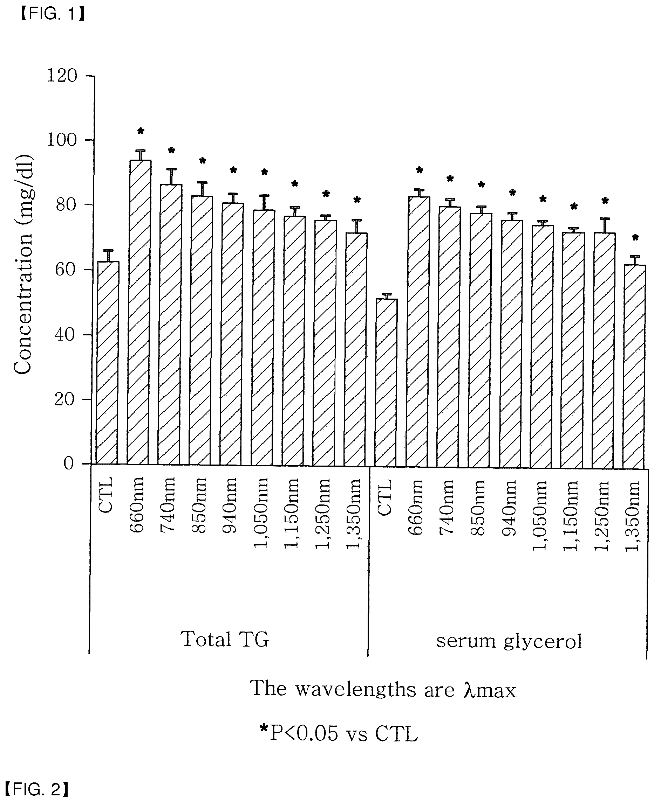

The result for Example 1 is shown in FIG. 1 and Table 1, and the result for Example 2 is shown in FIG. 2 and Table 2. In Table 1 and Table 2, the result for each irradiation wavelength is given as relative percentage (%) with respect to that of the control group (CTL) as 100%. In FIG. 1, FIG. 2, Table 1 and Table 2, the wavelength values are λₘₐₓ's.

**[Table 1]**

| | | |
|---|---|---|
| Total TG (%) | CTL | 100.0% |
| | 660 nm | 150.4% |
| | 740 nm | 138.4% |
| | 850 nm | 132.8% |
| | 940 nm | 129.6% |
| | 1,050 nm | 126.4% |
| | 1,150 nm | 123.2% |
| | 1,250 nm | 121.6% |
| | 1,350 nm | 115.2% |
| Serum glycerol (%) | CTL | 100.0% |
| | 660 nm | 160.6% |
| | 740 nm | 154.8% |
| | 850 nm | 151.0% |
| | 940 nm | 147.1% |
| | 1,050 nm | 144.2% |
| | 1,150 nm | 140.4% |
| | 1,250 nm | 140.4% |
| | 1,350 nm | 121.2% |

**[Table 2]**

| | | |
|---|---|---|
| Total TG (%) | CTL | 100.0% |
| | 660 nm | 219.8% |
| | 740 nm | 200.0% |
| | 850 nm | 185.4% |
| | 940 nm | 177.1% |
| | 1,050 nm | 168.8% |
| | 1,150 nm | 160.4% |
| | 1,250 nm | 156.3% |
| | 1,350 nm | 139.6% |
| Serum glycerol (%) | CTL | 100.0% |
| | 660 nm | 333.3% |
| | 740 nm | 311.1% |
| | 850 nm | 296.3% |
| | 940 nm | 281.5% |
| | 1,050 nm | 270.4% |
| | 1,150 nm | 255.6% |
| | 1,250 nm | 255.6% |
| | 1,350 nm | 181.5% |

As can be seen from FIG. 1 and Table 1, a lipolytic effect was achieved at all wavelengths when the cells were irradiated with light throughout the whole stages of differentiation. In particular, the highest lipolytic effect was achieved when the cells were irradiated with light with λₘₐₓ of 660 nm (the amount of eluted triglycerides was increased to 150.4% as compared to the control group and the amount of eluted glycerol was increased to 160.6% as compared to the control group). Even when light with λₘₐₓ of 1,350 nm was irradiated, at which the lowest lipolytic effect was achieved, the amount of eluted triglycerides was 115.2% as compared to the control group and the amount of eluted glycerol was 121.2% as compared to the control group. All of these results were statistically significant.

Also, as can be seen from FIG. 2 and Table 2, a lipolytic effect was achieved when at all wavelengths when the cells were irradiated with light after differentiation had been completed. In particular, the highest lipolytic effect was achieved when the cells were irradiated with light with λₘₐₓ of 660 nm (the amount of eluted triglycerides was increased to about 219.8 % as compared to the control group and the amount of eluted glycerol was increased to 333.3% as compared to the control group). Even when light with λₘₐₓ of 1,350 nm was irradiated, at which the lowest lipolytic effect was achieved, the amount of eluted triglycerides was 139.6% as compared to the control group and the amount of eluted glycerol was 181.5% as compared to the control group, which was statistically significant. Accordingly, it was confirmed that the irradiation of light according to the present disclosure to differentiated adipocytes also provides a very remarkable lipolytic effect. All of these results were statistically significant.

### [Test Example 2] Measurement of triglycerides remaining in cells

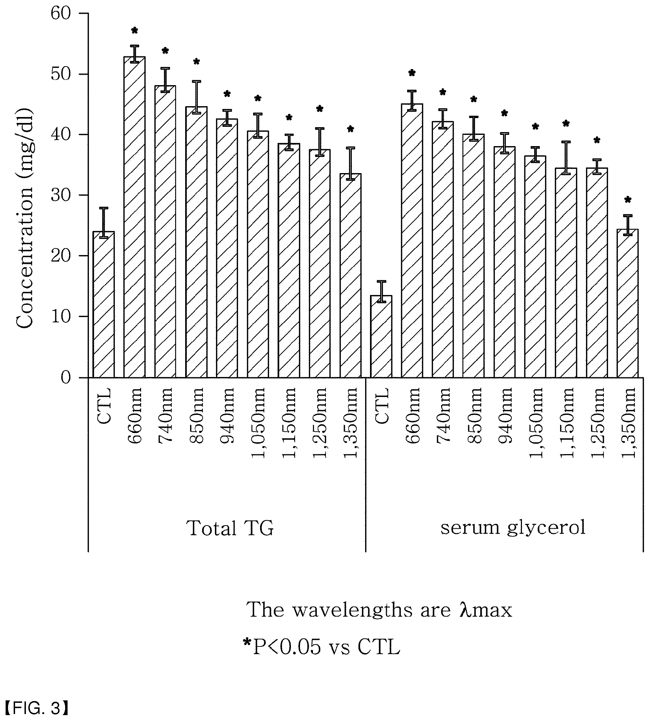

The cells remaining after the nutrient-restricted medium was recovered in Test Example 1 were fixed at room temperature by treating with a 3.7% formaldehyde solution (Sigma-Aldrich, St. Louis, USA) for 24 hours. Then, the cells were stained for 1 hour by adding 1 mL of a solution in which 0.5 g of Oil Red O (ORO; Sigma-Aldrich, St. Louis, USA) dissolved in 100 mL of propylene glycol (Sigma-Aldrich, St. Louis, USA). After removing the residual dye from the ORO-stained cells and washing 3 times with distilled water, followed by addition of 500 mL of isopropyl alcohol (IPA; Sigma-Aldrich, St. Louis, USA), the dye was extracted from the stained cells for 1 hour. The absorbance of the ORO-extracted IPA was measured at 490 nm. The result is shown in FIG. 3 and Table 3. In Table 3, the result is given as relative percentage (%) with respect to that of the control group (CTL) as 100%. In Table 3 and FIG. 3, the wavelength values are λₘₐₓ's.

**[Table 3]**

| | | |
|---|---|---|
| TG contents (%) | CTL | 100.0% |
| | 660 nm | 50.3% |
| | 740 nm | 57.0% |
| | 850 nm | 59.6% |
| | 940 nm | 62.2% |
| | 1,050 nm | 62.2% |
| | 1,150 nm | 66.8% |
| | 1,250 nm | 69.9% |
| | 1,350 nm | 79.8% |

As can be seen from FIG. 3 and Table 3, a lipolytic effect was confirmed because the triglycerides remaining in the differentiated adipocytes was decreased at all wavelengths. When light with λₘₐₓ of 660 nm was irradiated, at which the highest lipolytic effect was achieved, the amount of the triglycerides remaining in the adipocytes was decreased to 50.3% as compared to the control group, which was statistically significant. Even when light with λₘₐₓ of 1,350 nm was irradiated, at which the lowest lipolytic effect was achieved, the amount of the triglycerides remaining in the adipocytes was decreased to 79.8% as compared to the control group, which was statistically significant. All of these results were statistically significant.

From the results of Test Examples 1 and 2, it was confirmed that irradiation of light with specific wavelength to differentiating or differentiated adipocytes provides a remarkable effect of degrading triglycerides in the adipocytes to glycerol and provides a remarkable effect of eluting the triglycerides in the adipocytes out of the cells. Accordingly, it can be seen that the light irradiation according to the present disclosure provides a lipolytic effect.

## Claims

1. A kit for lipolysis comprising:
a light irradiation device which irradiates light; and
an instruction which describes a method of degrading fat by irradiating light,
wherein the light has a wavelength which is longer than 600 nm and equal to or shorter than 1500 nm.

2. The kit according to claim 1, wherein the light has a pattern which enhances skin penetration.

3. The kit according to claim 1, wherein the instruction comprises light irradiation intensity, light irradiation time, the distance between the irradiation device and skin, irradiation method and irradiated part.

4. The kit according to claim 3, wherein the light irradiation intensity is 1-600 J/cm².

5. The kit according to claim 3, wherein the light irradiation time is from 0.5 minute to 6 hours.

6. The kit according to claim 3, wherein the distance between the irradiation device and skin is 0-200 cm.

7. The kit according to claim 3, wherein the irradiation is performed by direct irradiation or using an auxiliary means that can be contacted to skin.

8. The kit according to claim 7, wherein the auxiliary means is one or more selected from a group consisting of a filter, a sheet, a film, a cosmetic, a gel and a cream.

9. The kit according to claim 3, wherein the irradiation is performed using one or more selected from a group consisting of a needle tube, a cannula and a microneedle.

10. The kit according to claim 3, wherein the irradiated part is any skin tissue where fat can be accumulated, excluding eyes, mouth, sexual organ and anus.

11. A slimming method comprising irradiating light to skin,
wherein the light has a wavelength which is longer than 600 nm and equal to or shorter than 1500 nm.

12. The slimming method according to claim 11, wherein the light has a pattern which enhances skin penetration.

13. The slimming method according to claim 11, wherein the light has an intensity of 1-600 J/cm².

14. The slimming method according to claim 11, wherein the irradiation is performed for from 0.5 minute to 6 hours.

15. The slimming method according to claim 11, wherein the irradiation is performed at a distance of 0-200 cm from skin.

16. The slimming method according to claim 11, wherein the irradiation is performed by direct irradiation or using an auxiliary means that can be contacted to skin.

17. The slimming method according to claim 16, wherein the auxiliary means is one or more selected from a group consisting of a filter, a sheet, a film, a cosmetic, a gel and a cream.

18. The slimming method according to claim 11, wherein the irradiation is performed using one or more selected from a group consisting of a needle tube, a cannula and a microneedle.

19. The slimming method according to any of claims 11 to 18, wherein the irradiation is performed onto the subcutaneous fat layer of skin.
